(19) Europäisches Patentamt
European Patent Office
Office européen des brevets

(11) Veröffentlichungsnummer: **0 354 500 A2**

(12) **EUROPÄISCHE PATENTANMELDUNG**

(21) Anmeldenummer: **89114497.4**

(22) Anmeldetag: **05.08.89**

(51) Int. Cl.4: **C08F 222/40 , C08G 73/12 , C07D 213/69 , C07D 237/16 , C07D 241/18 , C07D 239/52**

(30) Priorität: **10.08.88 DE 3827120**

(43) Veröffentlichungstag der Anmeldung: **14.02.90 Patentblatt 90/07**

(84) Benannte Vertragsstaaten: **CH DE ES FR GB IT LI NL**

(71) Anmelder: **BASF Aktiengesellschaft**
**Carl-Bosch-Strasse 38**
**D-6700 Ludwigshafen(DE)**

(72) Erfinder: **Eisenbarth, Philipp, Dr.**
**Gutleutstrasse 12**
**D-6702 Bad Duerkheim(DE)**
Erfinder: **Linden, Gerd, Dr.**
**Brunnengasse 18**
**D-6900 Heidelberg(DE)**
Erfinder: **Altstaedt,Volker,Dr.**
**Marienbaderstrasse 2**
**D-6084 Gernsheim(DE)**
Erfinder: **Peter, Roland, Dr.**
**Pfalzring 92**
**D-6704 Mutterstadt(DE)**

(54) **Hitzehärtbare Bismaleinimid-Formmassen.**

(57) Die Erfindung betrifft hitzehärtbare Bismaleinimid-Harze, die ein Bismaleinimid und ein heterocyclisches Comonomeres mit Alkenyl- oder Alkenyloxy-Endgruppen enthalten. Bevorzugtes Comonomer ist 2,6-Bis(2-propenylphenoxy)pyridin. Die Bismaleinimidharze sind zur Herstellung von Hochleistungsverbundwerkstoffen geeignet.

EP 0 354 500 A2

Xerox Copy Centre

## Hitzehärtbare Bismaleinimid-Formmassen

Die Erfindung betrifft hitzehärtbare Harze auf Basis von Bismaleinimiden.

Bismaleinimidharze, die aromatische Diamine als Comonomere enthalten, weisen zwar nach dem Aushärten selbst bei Temperaturen über 200 °C hervorragende mechanische und elektrische Eigenschaften auf, sie haben jedoch eine geringe Löslichkeit in üblichen Lösungsmitteln und ihre Aushärtungsprodukte sind sehr spröde.

Ein weiterer Nachteil sind ihre relativ hohen Erweichungstemperaturen, verbunden mit hohen Schmelzviskositäten; Halbzeuge bzw. Prepregs, hergestellt aus diesen Harzen, besitzen nicht die vom Verarbeiter oft gewünschte Klebrigkeit.

Niedrigere Viskositäten werden durch den Einsatz von zweikernigen Allylphenolen oder veretherten Allylphenolen erreicht, wie z.B. in der US-A 4 100 140 beschrieben. Infolge der relativ kurzen Kettenlänge und des daraus resultierenden relativ großen Anteils an aliphatischen Strukturelementen besitzen diese Harze jedoch eine für viele Anwendungen nicht ausreichende thermooxidative Beständigkeit. Darüber hinaus ist zur Erreichung von kurzen Härtungszeiten der Einsatz von Katalysatoren, z.B. von tertiären Aminen, unumgänglich; die vom Verarbeiter gewünschte Klebrigkeit ("tack") der entsprechenden Halbzeuge ist häufig nicht ausreichend.

Bismaleinimidharze, die mehrkernige aromatische Comonomere mit Alkenylenendgruppen enthalten, werden in der EP 230 741 beschrieben. Die dort eingesetzten Comonomeren führen entweder wegen des zu hohen aliphatischen Anteils zu Harzen mit nicht genügend hoher thermooxidativer Beständigkeit (Comonomere des Typs IIa nach EP 230 741) oder zu Harzen mit relativ hohen Erweichungspunkten bzw. Schmelzviskositäten (Comonomere des Typs IIb-e nach EP 230 741). Zur Beschleunigung der Härtung wird der Zusatz von Katalysatoren empfohlen.

Der Erfindung lag daher die Aufgabe zugrunde, Bismaleinimidharze bereitzustellen, welche zumindest partiell die genannten Nachteile nicht aufweisen.

Diese Aufgabe wird durch die erfindungsgemäßen Harze, welche heterocyclische Comonomere enthalten, gelöst. Sie haben bei Raumtemperatur eine hervorragende Klebrigkeit, verbunden mit einer niedrigen Viskosität. Auf grund ihrer stickstoffhaltigen Molekülstruktur härten diese Harze auch ohne Zusatz von Katalysatoren bei höheren Temperaturen rasch aus und ergeben vernetzte Polymere mit hoher Hitzebeständigkeit, guter Zähigkeit und relativ geringer Wasseraufnahme. Das günstige Viskositätsverhalten erlaubt die Anwendung von modernen Verarbeitungsverfahren, wie z.B. Filament-Winding oder Resin-Transfer-Moulding.

Die erfindungsgemäß einsetzbaren, heterocyclischen Comonomeren besitzen die allgemeine Formel I

R-Ar-O-Het-O-Ar-R I,

wobei der Rest R eine Alkenylgruppe mit 3 bis 6 C-Atomen oder eine entsprechende Alkenyloxygruppe bedeutet. Bevorzugt sind Allyl-, Propenyl- oder Allyloxygruppen.

Der Rest Ar bedeutet einen ein- oder zweikernigen aromatischen Rest, z.B. Phenylen, Naphthylen oder Phenylen-X-Phenylen (X ist eine chemische Bindung oder eine zweiwertige Gruppe, z.B. O, S, $SO_2$, CO oder $C(R^1)_2$ mit $R^1$ = H, $C_1$-$C_6$-Alkyl, Aryl, $CF_3$). Bevorzugt sind o-Phenylen, m-Phenylen und p-Phenylen.

Der Rest Ar kann zusätzlich zur Alkenylgruppe R noch eine OH-Gruppe besitzen.

Der heterocyclische Rest Het stellt einen stickstoffhaltigen, aromatischen Sechsring dar, ausgewählt aus der Gruppe

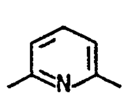 , 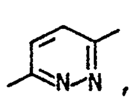 , 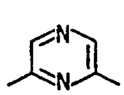 und 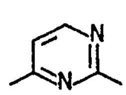 .

Bevorzugt sind der Pyridin- und der Pyridazinring.

Zur Herstellung der erfindungsgemäß einsetzbaren Comonomeren wird ein Dihalogenheterocyclus Hal-Het-Hal mit 2 Äquivalenten eines Bisphenols HO-Ar-OH in Gegenwart einer Base nach bekannten Methoden, z.B. nach DE-A 19 34 889, unter Bildung der heterocyclischen Phenole II kondensiert.

HO-Ar-O-Het-O-Ar-OH II

Einsetzbare Dihalogenheterocyclen sind 2,6-Dichlorpyridin, 3,6-Dichlorpyridazin, 2,6-Dichlorpyrazin und 2,6-Dichlorpyrimidin. Geeignete Bisphenole sind z.B. Hydrochinon, Resorcin oder Bisphenol A; weitere sind in DE-A 19 34 889 aufgeführt.

Die heterocyclischen Bisphenole II werden anschließend z.B. nach DE-A 28 18 091 mit einem

Alkenylhalogenid veräthert. Bevorzugte Alkenylhalogenide sind Allylchlorid, Allylbromid und Methallylchlorid.

Daran kann sich eine Claisen-Umlagerung, ebenfalls nach DE-A 28 18 091, anschließen, wobei man die entsprechenden OH-gruppenhaltigen Comonomeren (ortho-Allylphenole) erhält; diese lassen sich ferner basenkatalysiert nach EP-A 14 816 in die ebenfalls erfindungsgemäßen Propenylphenole überführen.

Auf diese Weise herstellbare Comonomere sind z.B. 2,6-Bis(3-allyl-4-hydroxyphenoxy)pyridin, 2,6-Bis(3-allyloxyphenoxy)pyridin, 2,6-Bis(4-allyl-3-hydroxyphenoxy)pyridin, 2,6-Bis[(3-allyl-4-hydroxyphenylisopropyl)phenoxy]pyridin sowie die entsprechenden Pyridazinderivate.

Eine alternative Methode zur Herstellung von Comonomeren der Formel I ist die Kondensation eines der beiden genannten Dihalogenheterocyclen Hal-Het-Hal mit 2 Äquivalenten eines Alkenylphenols HO-Ar-R nach bekannten Methoden. Einsetzbare Alkenylphenole sind z.B. 2-Allylphenol, 2-Propenylphenol und Eugenol.

Ein auf diese Weise herstellbares, bevorzugt einsetzbares Comonomer ist 2,6-Bis(2-propenylphenoxy)-pyridin. Diese heterocyclische Verbindung ist neu.

Die erfindungsgemäßen Bismaleinimidharze werden erhalten durch Umsetzung der heterocyclischen Alkenylverbindung mit einem Bismaleinimid der allgemeinen Formel

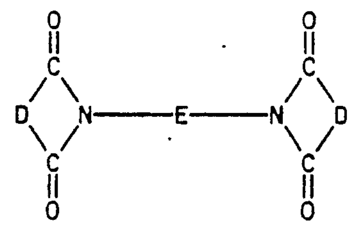
,

in der D eine gegebenenfalls substituierte Kohlenstoffdoppelbindung ist und E einen zweiwertigen Rest mit zumindest zwei Kohlenstoffatomen bedeutet. Bismaleinimide sind z.B. aus DE-A-2 040 094, DE-A-2 719 903 und DE-A-3 247 058 bekannt. Neben Bismaleinimiden sind grundsätzlich auch Polymaleinimide sowie Mischungen verschiedener Bismaleinimide geeignet. Bevorzugte Bismaleinimide sind 4,4′-Bismaleinimidodiphenylmethan, 4,4′-Bismaleinimidodiphenylether, 3,3′-Bismaleinimidodiphenylsulfon, 1,3-Bis maleinimidobenzol, 2,4-Bismaleinimidotoluol, 1,6-Bismaleinimidohexan und 2,2,4-Trimethyl-1,6-bismaleinimidohexan. Es können auch bis zu 20 Gew.% eines Monoimids enthalten sein.

Zur Herstellung der erfindungsgemäßen Bismaleinimidharze werden die Ausgangsmaterialien unter Anwendung der üblichen Techniken vermischt und auf Temperaturen zwischen 70 - 190 °C erhitzt, wobei die Bildung eines Präpolymeren erfolgt. Je nach Fortschritt der Vorpolymerisation erhält man eine relativ niedrig viskose Schmelze oder einen glasartig erstarrten Feststoff, der je nach Verwendungszweck gemahlen oder in einem Lösungsmittel gelöst wird. Die Herstellung der Harze kann auch in einem Lösungsmittel erfolgen.

Das Mischungsverhältnis bei der Umsetzung des Bismaleinimids mit der heterocyclischen Alkenylverbindung ist relativ frei wählbar, wobei ein Äquivalent-Verhältnis von 1 zu 0,05 bis 5 vorzuziehen ist.

Je nach beabsichtigtem Verwendungszweck kann es vorteilhaft sein, den erfindungsgemäßen Harzen weitere Komponenten beizufügen. In Frage kommende Zusatzkomponenten sind beispielsweise Amine, bevorzugt aromatische Diamine (z.B. 4,4′-Diaminodiphenylmethan) und Aminophenole, die ebenfalls eine Additionsreaktion mit den Maleinimiddoppelbindungen eingehen können. Es können auch Präpolymere, z.B. aus einem Bisimid und einem Amin eingesetzt werden.

Für bestimmte Anwendungen kann es auch zweckmäßig sein, zur Einstellung einer gewünschten Viskosität geeignete Vinylmonomere, z.B. Styrol, α-Methylstyrol, Divinylbenzol, Acryl- oder Methacrylsäureester, Diallylphthalat, 3,3′-Diallylbisphenol A, Triallylisocyanurat, Triallylcyanurat oder Vinylpyrrolidon einzusetzen. Ihr Anteil kann bis zu 50 Gew.%, bezogen auf die Mischung, betragen.

Die erfindungsgemäßen Mischungen können als weitere Zusätze Inhibitoren enthalten. Geeignete Inhibitoren sind Hydrochinon, Benzochinon oder Phenothiazin. Die Menge an eingesetzten Inhibitoren soll etwa zwischen 0,05 bis 1,5 Gew.% liegen.

Die erfindungsgemäßen Mischungen können weitere Zusätze, die in der Technologie der härtbaren Kunststoffe üblich sind, enthalten, wie Füllstoffe, Weichmacher, Pigmente, Farbstoffe, Formtrennmittel, flammhemmende Stoffe. Als Füllstoffe können auch Glas- und Kohlenstoffasern, Graphitpulver, Glimmer, Quarzpulver, Koalin oder Metallpulver bis zu einem Anteil von 80 Gew.%, bezogen auf die Mischung, verwendet werden.

Die erfindungsgemäßen Mischungen sind als Imprägnier-, Gieß- und Laminierharze oder als Formmassen (gefüllt oder ungefüllt) einsetzbar.

Dienen sie zur Herstellung von Hochleistungsverbundwerkstoffen, so kann die Tränkung von Glas-, Kohlenstoff- oder Aramidfasern unter Bildung von unidirektionalen oder Gewebeprepregs entweder aus der Schmelze bei 50 bis 150 °C oder aus Lösung erfolgen. Geeignete Lösungsmittel sind halogenierte Kohlenwasserstoffe, wie z.B. Dichlormethan, Ketone wie z.B. Aceton oder Methylethylketon, Glykolester, Toluol, Dimethylformamid, N-Methylpyrrolidon bzw. Gemische aus mehreren Lösungsmitteln.

Die Härtung der erfindungsgemäßen Harze erfolgt bei Temperaturen von ca. 100 bis 300 °C, gegebenenfalls unter Druck, vorzugsweise zwischen 160 bis 260 °C. Die gewählte Härtungstemperatur hängt entscheidend von der Länge der Härtungszeit ab und umgekehrt. Vielfach ist eine stufenweise Härtung vorteilhaft, wobei bei einer niedrigeren Temperatur zunächst unter Formgebung eine Vernetzung der Polymeren herbeigeführt wird. Nach Entformen kann sich dann zur vollständigen Aushärtung eine unter Umständen mehrstündige Nachhärtung oberhalb 200 °C anschließen.

Aus den erfindungsgemäßen Harzen können Hochleistungswerkstoffe, z.B. Isoliermaterialien, Strukturbauteile, Apparategehäuse und elektrische Bauteile hergestellt werden, die hohen Temperaturen ausgesetzt sind.

Beispiel 1

a) Herstellung von 2,6-Bis(2-propenylphenoxy)phenoxy)pyridin

Eine Mischung aus 296 g 2,6-Dichlorpyridin, 537 g 2-Allylphenol, 304 g Kaliumcarbonat, 2000 ml N-Methylpyrrolidon und 400 ml Chlorbenzol wurde 21 Stunden auf 190 °C erhitzt, wobei das entstehende Reaktionswasser azeotrop abdestilliert wurde. Anschließend wurde die Reaktionsmischung mit ca. 4 l Wasser versetzt und mit 4 l Dichlormethan extrahiert. Die vereinigten organischen Phasen wurden in einem Flüssig-Flüssig-Extraktor mit Wasser gewaschen, über Natriumsulfat getrocknet und im Vakuum eingedampft. Man erhielt 601,9 g (88%) Produkt als bräunliches Öl, das bei längerem Stehen langsam kristallisiert (farblose Kristalle mit Schmp. 69-73 °C); 1H-NMR:1,75 (d, 6H, $CH_3$), 5,60-5,80 (m, olef. H, kleiner Anteil an cis-Isomer), 6,20-6,50 (m, 6H, olef. und arom. H), 7,00-7,80 (m, 9H, arom.).

b) Herstellung eines Bismaleinimidharzes

Unter Rühren wurden 371 g Compimide 353 (monomerenhaltiges Bismaleinimidharz, Handelsprodukt der Fa. Technochemie Verfahrenstechnik, Dossenheim) und 200 g 2,6-Bis(2-propenylphenoxy)pyridin bei 140 °C zusammengeschmolzen, weitere 15 Min. erhitzt und dann wie folgt weiterverarbeitet:

Ein Teil des Harzes wurde zwecks schnelleren Abkühlens auf eine Metallfolie ausgegossen. Das Harz war bei Raumtemperatur sehr klebrig und besaß bei 75 °C eine Viskosität von 2320 mPas. Die Gelzeit betrug bei 160 °C 25 Min.

Der restliche Teil des Harzes wurde in 30x30x0,4 bzw. 0,1 cm große Metallformen gegossen und je 2 Stunden bei 160 °C und bei 190 °C sowie 10 Stunden bei 240 °C gehärtet. Das Polymer besaß bei 282 °C noch 50% seines Raumtemperatur-Schubmoduls (25 °C: 1400 $N/mm^2$; gemessen nach DIN 53 445). Der E-Modul betrug 3.600 $N \cdot mm^{-2}$ (nach DIN 53 457), die Schlagzähigkeit 8,2 $kJ \cdot m^{-2}$ (nach DIN 53 453). Nach 8-tägiger Lagerung in 70 °C heißem Wasser betrug die Wasserabsorption 4,21%.

c) Herstellung eines unidirektionalen Prepregs bzw. Laminats

Zur Herstellung eines unidirektionalen Prepregs mit der Kohlenstoff-Faser Torayca T 800 (Fa. Toray, Japan) wurde das Bismaleinimidharz nach 1b) bei ca. 65 °C zunächst auf ein kontinuierlich fortlaufendes Prepregpapier unter Bildung eines ca. 60 µm dicken Harzfilms aufgebracht und anschließend das Faserband mit einem Flächengewicht von 135 $g/m^2$ bei ca. 80 °C unter Druck in den Harzfilm eingelegt und vollständig getränkt. Der Harzgehalt des auf diese Weise erhaltenen Prepregs betrug 35 %, entsprechend einer nominellen Prepregdicke von 0,125 mm, bezogen auf 60 Vol.% Fasergehalt.

Das Prepreg besaß eine sehr gute Klebrigkeit ("Tack") und war leicht drapierbar; seine Harz-Glastemperatur betrug 4 °C (per DSC).

Zur Herstellung eines unidirektional verstärkten Hochleistungsverbundwerkstoffs wurden durch Übereinanderschichtung mehrerer Prepreglagen (entsprechend der Anforderung durch die Prüfkörpernorm) Laminate hergestellt und mit dem unter 1b) angegebenen Härtungscyclus gehärtet. Das Laminat besaß einen Glc-Wert von 262 kJ/$m^2$ (nach NASA-Prüfnorm RP 10920).

Beispiel 2

a) Herstellung von 2,6-Bis(3-allyloxyphenoxy)pyridin

Zur Lösung von 87,9 g 2,6-Bis(3-hydroxyphenoxy)pyridin (hergestellt nach DE-A 19 34 889 aus 2,6-Dichlorpyridin + 2 Mol Resorcin; farbl. Kristalle mit Fp. 188° C) und 26,4 g Natriumhydroxid in 500 ml n-Propanol wurden bei 95° C 59,7 g Allylchlorid zugetropft. Man erhitzte weitere 10 Stunden unter Rückfluß, gab nach Abkühlen auf Raumtemperatur 500 ml Dichlormethan hinzu und filtrierte das ausgefallene Natriumchlorid ab. Nach Abdestillieren des Lösungsmittels im Vakuum erhielt man 101 g (90%) Produkt als gelbliches Öl mit einer Viskosität von 60 mPas bei 50° C; NMR(300 MHZ, D6-DMSO): 4.56 (d, 4H, allyl), 5.22-5.42 (dd, 4H, allyl), 5.95-6.10 (m, 2H, allyl), 6.60-6,85 (m, 6H, arom.), 7.22 (t, 2H, arom.), 7.82 (t, 1H, arom.).

b) Herstellung von 2,6-Bis[4(2)-allyl-3-hydroxyphenoxy]pyridin

64 g Allylether aus Beispiel 2a) wurden 5 Stunden auf 190° C erhitzt. Ausbeute: 63 g (97%).

c) Herstellung eines Bismaleinimidharzes

Analog Beispiel 1 wurde aus 70 g 4,4'-Bismaleinimidodiphenylmethan und 30 g Allylether aus Beispiel 2b) ein Bismaleinimidharz hergestellt, das bei Raumtemperatur sehr klebrig war und bei 75° C eine Viskosität von 2100 mPas besaß. Nach Härtung analog Beispiel 1 wurde ein Polymer erhalten, das bei 290° C noch 50% seines Raumtemperatur-Schubmoduls besaß (25° C : 1500 N/$mm^2$; nach DIN 53 445).

Beispiel 3

a) Herstellung von 3,6-Bis(2-propenylphenoxy)pyridazin

Eine Mischung von 149 g 3,6-Dichlorpyridazin, 268,4 g 2-Allylphenol, 152 g Kaliumcarbonat, 400 ml N-Methylpyrrolidon und 900 ml Toluol wurde 30 Stunden auf 140° C erhitzt, wobei das entstehende Reaktionswasser azeotrop abdestilliert wurde. Nach Aufarbeitung analog zu Beispiel 1a) erhielt man 317 g (92 %) eines teilweise kristallisierten Produkts, das aus einem Isomerengemisch zwischen allyl- und propenylgruppenhaltigem Produkt bestand. Nach Zugabe von Methanol und Abfiltrieren wurden 140 g reines 3,6-Bis(2-propenylphenoxy)pyridazin als farblose Kristalle mit Schmelzpunkt 165-168° C gewonnen; 1H-NMR: 1.80 (d, CH3), 5.70-5.90 (m, kleiner Anteil an cis-Isomer), 6.20-6.50 (m, 4H, olef.), 7.0-7.7 (m, 10H, arom.).

b) Herstellung eines Bismaleinimidharzes

Es wurde analog Beispiel 1a) ein Harz aus 60 g Compimide 353, 40 g 3,6-Bis(2-propenylphenoxy)-pyridazin und 15 g Diallylphthalat hergestellt und weiterverarbeitet; das Harz besaß eine Viskosität von 3600 mPas und war bei Raumtemperatur sehr klebrig. Nach Härtung analog Beispiel 1 wurde ein Polymer erhalten, das bei 283° C noch 50% seines Raumtemperatur-Schubmoduls besaß (25° C : 1350 N/$mm^2$; nach DIN 53 445).

## Ansprüche

1. Hitzehärtbare Bismaleinimid-Harze, enthaltend

A. ein Bismaleinimid,

B. ein heterocyclisches Comonomer der Formel

R-Ar-O-Het-O-Ar-R I,

wobei die Symbole folgende Bedeutung haben:

R ist eine Alkenyl- oder Alkenyloxy-Gruppe mit 3 bis 6 Kohlenstoffatomen,

Ar ist ein Phenylen-, Naphthylen- oder

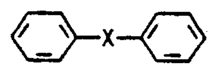

-Rest

mit X = O, S, $SO_2$, CO, $C(R')_2$ ($R^1$ = H, $C_1$-$C_6$-Alkyl, $CF_3$ oder Phenyl) oder eine chemische Bindung, wobei Ar gegebenenfalls eine Hydroxylgruppe tragen kann,

Het ist ein heterocyclischer Sechsring, ausgewählt aus der Gruppe

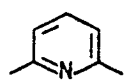 , 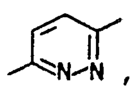 , 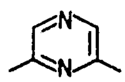 und 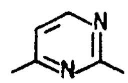 .

2. Hitzehärtbare Bismaleinimidharze nach Anspruch 1, dadurch gekennzeichnet, daß ein heterocyclisches Comonomer I eingesetzt wird, in dem R Propenyl, Ar 1,2-Phenylen und Het ein 2,6-Pyridin-Rest ist.

3. Heterocyclische Verbindung der Formel I, in welcher R Propenyl, Ar 1,2-Phenylen und Het ein 2,6-Pyridin-Rest ist.

4. Verwendung der Bismaleinimidharze nach Anspruch 1 zur Herstellung von Hochleistungsverbundwerkstoffen mit Glas- oder Kohlenstoffasern.